# FASCICULE DE BREVET EUROPEEN

(11) **EP 1 406 591 B1**
(45) Date de publication et mention de la délivrance du brevet: **07.03.2007**
(21) Numéro de dépôt: 02770036.8
(22) Date de dépôt: 18.07.2002
(51) Int. Cl.: A61K 9/08, A61K 31/565, A61K 31/57

(54) **COMPOSITION PHARMACEUTIQUE POUR L'ADMINISTRATION PAR VOIE NASALE D'ESTRADIOL ET DE NORETHISTERONE**
PHARMAZEUTISCHE ZUSAMMENSETZUNG FÜR DIE NASALE VERABREICHUNG VON ÖSTRADIOL UND NORETHISTERON
PHARMACEUTICAL COMPOSITION FOR NASAL DELIVERY OF ESTRADIOL AND NORETHISTERONE

(30) Priorité: 19.07.2001 FR 0109654
(43) Date de publication de la demande: 14.04.2004
(73) Titulaire: Les Laboratoires Servier, 92415 Courbevoie Cédex (FR)
(72) Inventeur: FONKNECHTEN, Gilles, F-45000 Orléans (FR); WUTHRICH, Patrick, F-45000 Orleans (FR); TSOUDEROS, Yannis, F-75014 Paris (FR); VARIN, Claire, F-78110 le Vesinet (FR)
(86) Numéro de dépôt international: PCT/FR2002/002561
(87) Numéro de publication internationale: WO 2003/015751

(56) Documents cités:
- EP-A- 0 349 091
- H. KUBLIK ET AL.: "Nasal absorption of 17-beta-estradiol from different cyclodextrin inclusion formulations in sheep" EUROPEAN JOURNAL OF PHARMACEUTICS AND BIOPHARMACEUTICS, vol. 42, no. 5, octobre 1996 (1996-10), pages 320-324, XP000632691 Stuttgart (DE)

## Description

La présente invention concerne une composition pharmaceutique pour l'administration par voie nasale du 17-β-estradiol et de noréthistérone.

Chez la femme, la carence oestrogénique est responsable à court terme des troubles climatériques chez 70 % des femmes, à plus long terme d'une ostéoporose chez 30 % d'entre elles et d'une augmentation du risque cardiovasculaire. Le traitement substitutif de la ménopause reste confronté à des difficultés de compliance qui n'ont qu'en partie été améliorées par les formes pharmaceutiques actuellement disponibles.

Les comprimés qui sont largement utilisés ont l'avantage de l'ancienneté et de la simplicité mais leurs dosages manquent de souplesse pour l'adaptation posologique. De plus, le phénomène de premier passage intestinal et hépatique est responsable de leurs inconvénients métaboliques. Les systèmes transdermiques permettent d'éviter de phénomène de premier passage mais présentent des inconvénients importants comme la mauvaise tolérance locale, la variabilité des doses délivrées et l'adaptation difficile des doses. La voie nasale permet une administration de principe actif à visée systémique en évitant le phénomène de premier passage hépatique tout en permettant une adaptation aisée des doses administrées par simple modification du nombre de pulvérisations.

Il est particulièrement intéressant d'associer dans la même formulation le 17-β-estradiol et un progestatif pour les raisons suivantes : l'adjonction d'un progestatif est indispensable chez les femmes non hystérectomisées pour s'opposer aux effets prolifératifs de l'estradiol sur l'endomètre et diminuer ainsi le risque d'hyperplasie et de cancer de l'endomètre. L'association dans la même formulation, assurant la prise effective du progestatif, garantit ainsi une bonne compliance et donc l'efficacité du traitement.

L'état antérieur de la technique est notamment illustré par le brevet EP 0 349 091 qui décrit une formulation destinée à l'administration nasale chez les femmes d'hormones sexuelles comme le 17-β-oestradiol ou la progestérone et contenant une cyclodextrine, ou par le brevet EP 0 689 442 qui décrit une composition nasale contenant un progestatif et une cyclodextrine.
Or, ces brevets décrivent de manière générale des formulations nasales contenant des hormones sexuelles et une cyclodextrine sans qu'aucune indication sur les quantités respectives des constituants ne soit donnée.

Dans ces formulations, la cyclodextrine par complexation des hormones permet leur solubilisation en milieu aqueux afin d'obtenir une solution limpide utilisable pour l'administration par voie nasale. Ces solutions doivent être stables et lors de l'administration par voie nasale, elles doivent permettre la libération d'une quantité d'hormones suffisante pour l'activité thérapeutique.

Le problème à résoudre est donc la mise au point d'une formulation nasale pour l'administration d'estradiol et de noréthistérone contenant une cyclodextrine dans laquelle la quantité de cyclodextrine permet la complexation des deux hormones afin d'obtenir une solution stable et compatible avec la physiologie nasale, permet une bonne absorption des hormones à travers la muqueuse nasale et une biodisponibilité suffisante des hormones pour conduire à l'effet thérapeutique souhaité.

Or, il s'est avéré que la mise au point d'une formulation nasale contenant à la fois le 17-β-estradiol, un progestatif et une cyclodextrine qui permette de répondre aux critères thérapeutiques était particulièrement complexe.

En effet, dans ces formulations nasales, l'absorption des hormones sexuelles à travers la muqueuse nasale n'est possible que si les hormones sont dissoutes au préalable dans la solution aqueuse à administrer.
D'autre part, il est indispensable dans ces solutions que non seulement les hormones se trouvent dans des quantités nécessaires pour obtenir l'effet thérapeutique souhaité mais telles que la décomplexation au moment du passage de la muqueuse nasale soit la plus complète possible afin d'éviter que ne soient pas absorbées de grandes quantités de produits complexés sans effet thérapeutique.

Enfin, il est également souhaitable dans ces formulations que la quantité de cyclodextrine soit la plus petite possible afin de ne pas avoir d'effets secondaires dus à cet excipient.

Or, ces hormones sont faiblement solubles en milieu aqueux et l'utilisation de complexants comme les cyclodextrines permet leur solubilisation. Cependant, dans des formulations nasales contenant deux hormones sexuelles comme le 17-β-estradiol et un progestatif, la formation des complexes s'est avérée ne pas être une simple addition des quantités de complexants pour chacun des deux principes actifs à complexer. Il a été montré de manière surprenante que l'équilibre est atteint pour une quantité inférieure du complexant.

D'autre part, l'adjonction d'une quantité supérieure de complexant par rapport à la quantité nécessaire minimale pour l'obtention d'une solution des produits complexés physiquement stables, conduit à une perte de biodisponibilité des deux principes actifs et donc à augmenter les doses en principes actifs afin d'obtenir l'effet thérapeutique souhaité. Or, il est indispensable de réduire la quantité de principes actifs au strict nécessaire pour obtenir l'activité recherchée.

Les documents de l'art antérieur cités précédemment ne permettaient en aucun cas de prévoir et de résoudre ces difficultés.

Plus spécifiquement, la présente invention concerne une composition pharmaceutique sous forme de solution aqueuse pour l'administration par voie nasale de 17-β-oestradiol sous forme éventuellement hydratée et de noréthistérone ou d'acétate de noréthistérone contenant une cyclodextrine partiellement méthylée caractérisée en ce quelle contient :
- une quantité d'estradiol comprise entre 2,2 et 2,8 mg par ml de solution,
- une quantité de noréthistérone ou d'acétate de noréthistérone comprise entre 3,5 et 4,5 mg par ml de solution,
- et une quantité de cyclodextrine partiellement méthylée comprise entre 60 et 70 mg par ml de solution.

A titre préférentiel, la composition selon l'invention contiendra une cyclodextrine méthylée partiellement et de manière randomisée dont le degré de substitution moyen par des groupements méthyles est voisin de 1,7 ci-après dénommée RAMEB.

Dans la composition selon l'invention, l'estradiol se trouve préférentiellement sous forme d'hémihydrate.

Dans la composition selon l'invention, le progestatif sera préférentiellement la noréthistérone.

Le pH de la solution aqueuse selon l'invention sera préférentiellement le pH physiologique obtenu par ajout d'une solution de chlorure de sodium permettant de maintenir l'isotonicité de la solution et ajustement éventuel à pH 6 pour mimer le pH nasal.

La composition préférée selon l'invention est une solution aqueuse pour l'administration par voie nasale de 17-β-estradiol et de noréthistérone (NET) contenant du RAMEB, caractérisée en ce qu'elle contient :
- 2,5 mg d'estradiol hémydrate par ml de solution,
- 3,93 mg de NET par ml de solution,
- 65 mg de RAMEB par ml de solution.

La composition pharmaceutique selon l'invention sera administrée au moyen d'un dispositif permettant l'administration par voie nasale délivrant à chaque pulvérisation la quantité nécessaire en principes actifs permettant d'obtenir l'effet thérapeutique souhaité. Cette solution nasale est préférentiellement conditionnée dans un flacon muni d'une pompe doseuse permettant d'obtenir des pulvérisations de 70 µl. Cette pompe doseuse délivrera à chaque pulvérisation des quantités de la solution nasale selon l'invention contenant pour 70 µl des quantités de principes actifs comprises entre 154 et 196 µg d'estradiol (hémihydrate) et 245 et 315 µg de noréthistérone.

### Exemple de composition pharmaceutique selon l'invention

Composition d'une solution aqueuse nasale selon l'invention pour 1 ml :

| | |
|---|---|
| Estradiol hémihydrate | 2,5 mg |
| Noréthistérone | 3,93 mg |
| RAMEB | 65 mg (6,5 %) |
| Chlorure de sodium | 9 mg |

Le pH de cette solution est égal à 6.

Cette composition a été testée cliniquement afin de déterminer les concentrations plasmatiques en estradiol et en noréthistérone en comparaison avec une solution contenant 75 mg de RAMEB (7,5 %) pour les mêmes quantités de principes actifs.

La courbe ci-dessous montre l'influence de la concentration en RAMEB sur la cinétique de l'estradiol :

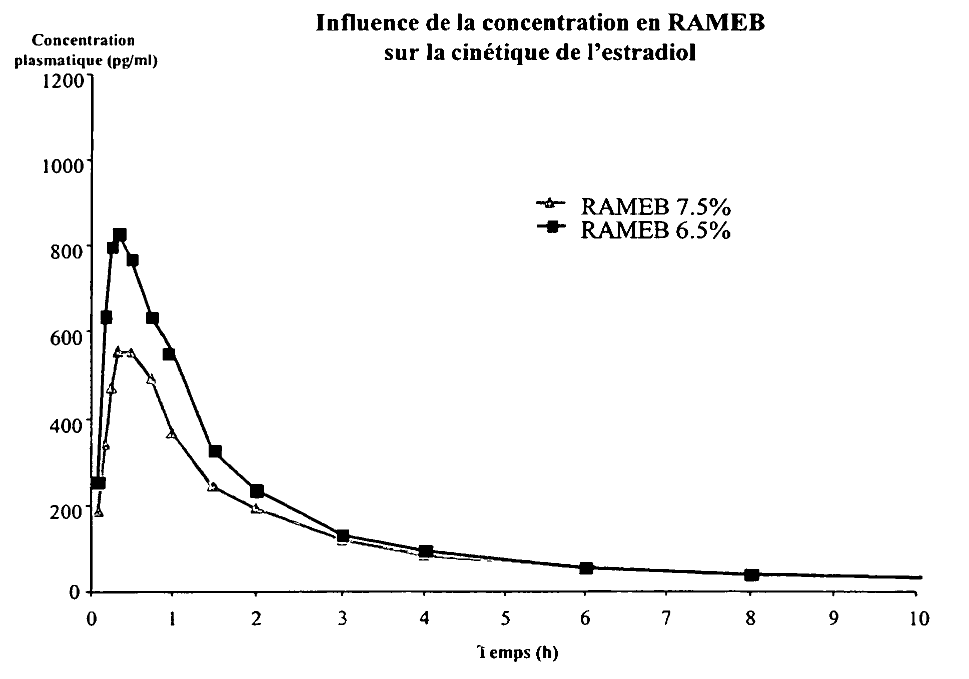

Ces résultats montrent que lorsque la concentration en RAMEB augmente (RAMEB 7,5 %), on observe une perte de biodisponibilité d'environ 30 % : la solution nasale selon l'invention qui contient 6,5 % de RAMEB permet d'obtenir une concentration plasmatique en estradiol conduisant à l'effet thérapeutique recherché alors qu'une solution nasale contenant 7,5 % de RAMEB conduit à une perte de biodisponibilité telle qu'elle n'est pas envisageable pour obtenir l'effet thérapeutique recherché.

Afin d'obtenir la biodisponibilité souhaitée de l'estradiol, une solution contenant 7,5% de RAMEB avec des quantités plus importantes en principes actifs a été préparée : cette solution s'est avérée inutilisable en raison d'une reprécipitation dans le temps des principes actifs.

Une autre solution nasale contenant les mêmes quantités en principes actifs mais une quantité de RAMEB égale à 55 mg par ml de solution a été préparée : cette solution s'est avérée inutilisable en raison d'une reprécipitation dans le temps des principes actifs.

Ces résultats ont également été observés sur la cinétique de la noréthistérone.

Ainsi, il est montré à travers ces exemples que seules les compositions aqueuses selon l'invention permettent d'obtenir non seulement des solutions stables, sans reprécipitation des principes actifs mais également des solutions assurant une biodisponibilité maximale en principes actifs conduisant à l'effet thérapeutique visé.

## Revendications

1. Composition pharmaceutique sous forme de solution aqueuse pour l'administration par voie nasale de 17-β-estradiol éventuellement sous forme hydratée et de noréthistérone ou d'acétate de noréthistérone contenant une cyclodextrine partiellement méthylée **caractérisée en ce qu'**elle contient :
- une quantité d'estradiol comprise entre 2,2 et 2,8 mg par ml de solution,
- une quantité de noréthistérone ou d'acétate de noréthistérone comprise entre 3,5 et 4,5 mg par ml de solution,
- et une quantité de cyclodextrine partiellement méthylée comprise entre 60 et 70 mg par ml de solution.

2. Composition pharmaceutique selon la revendication 1 **caractérisée en ce que** l'estradiol est sous forme d'hémihydrate.

3. Composition pharmaceutique selon la revendication 1 **caractérisée en ce qu'**elle contient de la noréthistérone (NET).

4. Composition pharmaceutique selon la revendication 1 **caractérisée en ce que** la cyclodextrine est partiellement méthylée et de manière randomisée dont le degré de substitution moyen des groupements méthyles est voisin de 1,7 (RAMEB).

5. Composition pharmaceutique selon l'une quelconque des revendications 1 à 4 **caractérisée en ce qu'**elle contient :
- 2,5 mg d'estradiol hémydrate par ml de solution,
- 3,93 mg de NET par ml de solution,
- 65 mg de RAMEB par ml de solution.

## Claims

1. Pharmaceutical composition in the form of an aqueous solution for administration, by the nasal route, of 17-β-oestradiol optionally in hydrated form, and norethisterone or norethisterone acetate, containing a partially methylated cyclodextrin, **characterised in that** it comprises:
- oestradiol in an amount of from 2.2 to 2.8 mg per ml of solution,
- norethisterone or norethisterone acetate in an amount of from 3.5 to 4.5 mg per ml of solution,
- and partially methylated cyclodextrin in an amount of from 60 to 70 mg per ml of solution.

2. Pharmaceutical composition according to claim 1, **characterised in that** oestradiol is in hemihydrate form.

3. Pharmaceutical composition according to claim 1, **characterised in that** it contains norethisterone (NET).

4. Pharmaceutical composition according to claim 1, **characterised in that** the cyclodextrin is randomly partially methylated, the average degree of substitution by the methylated groups being approximately 1.7 (RAMEB).

5. Pharmaceutical composition according to any one of claims 1 to 4, **characterised in that** it contains :
- 2.5 mg of oestradiol hemihydrate per ml of solution,
- 3.93 mg of NET per ml of solution,
- 65 mg of RAMEB per ml of solution.

## Patentansprüche

1. Pharmazeutische Zubereitung in Form einer wässrigen Lösung zur Verabreichung auf nasalem Wege von 17β-Oestradiol gegebenenfalls in Form seines Hydrats, und von Norethisteron oder von Norethisteronacetat enthaltend ein teilweise methyliertes Cyclodextrin, **dadurch gekennzeichnet, daß** sie:
- eine Menge von Oestradiol zwischen 2,2 und 2,8 mg pro ml der Lösung,
- eine Menge von Norethisteron oder Norethisteronacetat zwischen 3,5 und 4,5 mg pro ml der Lösung, und
- eine Menge von teilweise methyliertem Cyclodextrin zwischen 60 und 70 mg pro ml der Lösung enthält.

2. Pharmazeutische Zubereitung nach Anspruch 1, **dadurch gekennzeichnet, daß** das Oestradiol in Form des Hemihydrats vorliegt.

3. Pharmazeutische Zubereitung nach Anspruch 1, **dadurch gekennzeichnet, daß** sie Norethisteron (NET) enthält.

4. Pharmazeutische Zubereitung nach Anspruch 1, **dadurch gekennzeichnet, daß** das Cyclodextrin teilweise und in der Weise statistisch methyliert ist, daß der der mittlere Substitutionsgrad der Methylgruppen etwa 1,7 beträgt (RAMEB).

5. Pharmazeutische Zubereitung nach einem der Ansprüche 1 bis 4, **dadurch gekennzeichnet, daß** sie:
- 2,5 mg Oestradiol-Hemihydrat pro ml der Lösung,
- 3,93 mg NET pro ml der Lösung, und
- 65 mg RAMEB pro ml der Lösung enthält.
